# EUROPEAN PATENT SPECIFICATION

(11) **EP 1 848 487 B1**
(45) Date of publication and mention of the grant of the patent: **19.01.2011**
(21) Application number: 06735346.6
(22) Date of filing: 16.02.2006
(51) Int. Cl.: A61M 25/10, A61M 25/00

(54) **Medical devices**
Medizinische Vorrichtungen
Dispositifs medicaux

(30) Priority: 17.02.2005 US 60151
(43) Date of publication of application: 31.10.2007
(73) Proprietor: Boston Scientific Limited, Christ Church (BB)
(72) Inventor: KLISCH, Leo, M., Maple Grove, Minnesota 55311 (US); WEBER, Jan, 6228 GJ Maastricht (NL); HOLMAN, Thomas, J., Princeton, MN 55371 (US); SCHEWE, Scott, R., Eden Prairie, Minnesota 55346-1334 (US); ALI, Afsar, Maple Grove, Minnesota 55311 (US); NODDIN, Richard, A., Elk River, Minnesota 55330 (US); SHIPPY, James Lee, III, Roswell, Georgia 30075 (US); JAGGER, Karl, Deephaven, Minnesota 55331 (US); SEPPALA, Jan, Maple Grove, Minnesota 55369 (US)
(74) Representative: Peterreins, Frank
(86) International application number: PCT/US2006/005641
(87) International publication number: WO 2006/089115

(56) References cited:
- WO-A-99/25417
- US-A- 5 336 234
- US-A- 5 423 745
- US-A- 5 826 588
- US-A1- 2001 035 456
- US-A1- 2002 072 707
- US-A1- 2003 055 378
- US-A1- 2003 163 148
- US-A1- 2004 086 674
- US-A1- 2005 015 107
- US-A1- 2005 177 130
- US-B1- 6 786 889

## Description

### TECHNICAL FIELD

The invention relates to medical devices, such as those that include balloons, and methods of making the devices.

### BACKGROUND

Medical devices that include balloons, such as balloon catheters, can be used to administer a variety of treatments. For example, in an angioplasty procedure, a balloon carried by a catheter can be used to widen a constricted bodily vessel, such as a coronary artery. A balloon catheter can also be used to deliver a tubular member, such as a stent, that is placed in the body to reinforce or to reopen a blocked vessel.

In angioplasty, the balloon can be used to treat a stenosis, or a narrowing of the bodily vessel, by collapsing the balloon and delivering it to a region of the vessel that has been narrowed to such a degree that blood flow is restricted. The balloon can be delivered to a target site by passing the catheter over an emplaced guidewire and advancing the catheter to the site. In some cases, the path to the site can be rather tortuous and/or narrow. Upon reaching the site, the balloon is then expanded, e.g., by injecting a fluid into the interior of the balloon. Expanding the balloon can expand the stenosis radially so that the vessel can permit an acceptable rate of blood flow. After use, the balloon is collapsed and withdrawn.

In stent delivery, the stent is compacted on the balloon and transported to a target site.

Upon reaching the site, the balloon can be expanded to deform and to fix the stent at a predetermined position, e.g., in contact with the vessel wall. The balloon can then be collapsed and withdrawn.

Medical balloons can be manufactured by extruding a cylindrical tube of polymer and then pressurizing the tube while heating to expand the tube into the shape of a balloon. The balloon can be fastened around the exterior of a hollow catheter shaft to form a balloon catheter. The hollow interior of the balloon is in fluid communication with the hollow interior of the shaft. The shaft may be used to provide a fluid supply for inflating the balloon or a vacuum for deflating the balloon.

U.S. 2003/0055378 discloses an inflatable medical balloon for multiple procedures, including angioplasty procedures, procedures for delivering medical devices, such as stents, and a method of making the catheter systems. The catheter system employs a balloon having a plurality of flexible portions and a combination of hard and soft portions.

WO 99/25417 discloses a balloon and method of manufacture for use in catheters for medical procedures, in particular percutaneous translumenal coronary angioplasty and other cardiovascular interventions. The balloon incorporates an inflatable membrane having a number of perpendicular ribs which have a higher resistance to collapse during deflation.

### SUMMARY

The invention relates to a method as disclosed in ony one of claims 1-16 and to a device as disclosed in any one of claims 17-25.

In on aspect, the medical device includes a medical balloon having a a body portion having a plurality of first regions and a plurality of second regions, the second regions having a greater thickness than the first regions, each of the first regions including a center that is substantially aligned with an axis about which the balloon can be folded.

Embodiments may include one or more of the following features.

In some embodiments, the first region comprises a laser ablated area of the cone portion.

In some embodiments, the balloon comprises a first layer and a second layer, the raised feature comprising the second layer.

In certain embodiments, the second layer is constructed of a material (e.g., Pebax 25D) having a hardness less than a hardness of the first layer (e.g., Pebax 72D).

In certain embodiments, the method includes applying a second material to the outer surface of the balloon.

In some embodiments, the second material is harder than the material of the body portion.

In certain embodiments, the method includes ablating regions of the second material to form cutting elements extending from the outer surface of the body portion.

In some embodiments, the second material is softer than the material of the body portion.

In some embodiments, the method includes removing at least a portion of the second material.

Embodiments may have one or more of the following advantages. The ability of the medical device to inflate and/or deflate is enhanced. The manner in which the medical device inflates and/or deflates can be controlled. The manner in which a medical device ruptures can be controlled. Foldability of the medical device is enhanced. Stent retention capability of the medical device is improved. The ability of the medical device to retain a cutting element is improved. Substantial uniformity of an outer surface of the medical device can be achieved. The medical device may have a reduce profile, which facilitates trackability and delivery into the body.

As used herein, a "body portion" of a balloon refers to the generally central portion of the balloon between the cone portions of the balloon. The body portion is typically the portion of the balloon with the largest width or diameter when the balloon is fully inflated. A "cone portion" of a balloon refers to a portion of the balloon that has a variable (e.g., tapered) width or diameter. A "waist portion" of a balloon refers to the portion of the balloon that contacts a portion of catheter shaft. The waist portion is typically the portion of the balloon with the smallest width or diameter. The waist portion, for example, can have an inner diameter that is substantially equal to the outer diameter of the catheter shaft.

Other aspects, features, and advantages will be apparent from the description of the embodiments thereof and from the claims.

### DESCRIPTION OF DRAWINGS

FIG 1A is a side view of an embodiment of a medical balloon having recessed channels extending along the balloon.
FIG 1B is a cross-sectional view of a cone portion of the balloon of FIG 1A taken along line 1B-1B.
FIG 1C is a cross-sectional view of the medical balloon of FIG. 1A in a deflated state.
FIG. 1D is a cross-sectional view of the medical balloon of FIG 1A in a partially folded state.
FIG 1E is a cross-sectional view of the medical balloon of FIG. 1A in a substantially completely folded state.
FIG 2 is a perspective view of an embodiment of a tube used to form a medical balloon.
FIG 3 is a perspective view of a medical balloon having tapered recessed regions in the cone portions of the balloon.
FIG 4 is a perspective view of a medical balloon having curved recessed regions in the cone portions of the balloon.
FIG. 5 is a perspective view of a medical balloon having recessed panels extending along the waist portions and the cone portions of the balloon.
FIG 6 is a perspective view of a medical balloon having recessed panels extending along the waist portions and the cone portions of the balloon.
FIG 7 is a cross-sectional view of the cone portion of the balloon of FIG 6 taken along line 7-7.
FIG 8 is a perspective view of medical balloon having a drug eluting layer and carrying a stent.
FIG 9 is a side view of a medical balloon having raised rings extending around the circumference of the balloon.
FIG 10 is a side view of a medical balloon having raised rings extending around the circumference of the balloon in a helical configuration.
FIG 11 is a side view of a medical balloon having multiple, cylindrical protrusions extending from the body portion of the balloon.
FIG 12 is a side view of a medical balloon having multiple, rectangular protrusions extending from the body portion of the balloon.
FIG 13 is a side view of a medical balloon having rectangular protrusions arranged in a lattice configuration expending from the body portion of the balloon.
FIG 14 is a perspective view of an embodiment of a medical balloon having cutting blades extending from the body portion of the balloon.
FIG 15 is a perspective view of another embodiment of a medical balloon having cutting blades extending from the body portion of the balloon.
FIG 16 is a side view of a medical balloon having an outer layer that tapers from a first region to a second region of the balloon.
FIG. 17 is a cross-sectional view of the medical balloon of FIG 16 taken along line 17-17.
FIG 18 is a cross-sectional view of a non-concentric parison.
FIG 19 is a perspective view of a balloon including rings surrounding the distal and proximal ends of the body portion of the balloon.
FIG 20 is a cross-sectional view of the balloon of FIG. 19 taken along line 20-20.
FIG 21 is a perspective view of a balloon including ribs extending along the cone portion of the balloon.

### DETAILED DESCRIPTION

In one aspect, the invention features a medical device, such as a medical balloon, formed by and/or manufactured by using laser ablation. For example, laser ablation can be used to enhance the ease with which a medical balloon can fold, to encourage a longitudinal rupture of a medical balloon in the case of failure, to enhance the ability of a medical balloon to retain a stent, and to create cutting blades on a medical balloon. In another aspect, as described below, laser ablation can also be used to form and/or to modify other medical devices, such as medical catheters.

### Balloon Folding and Rupture

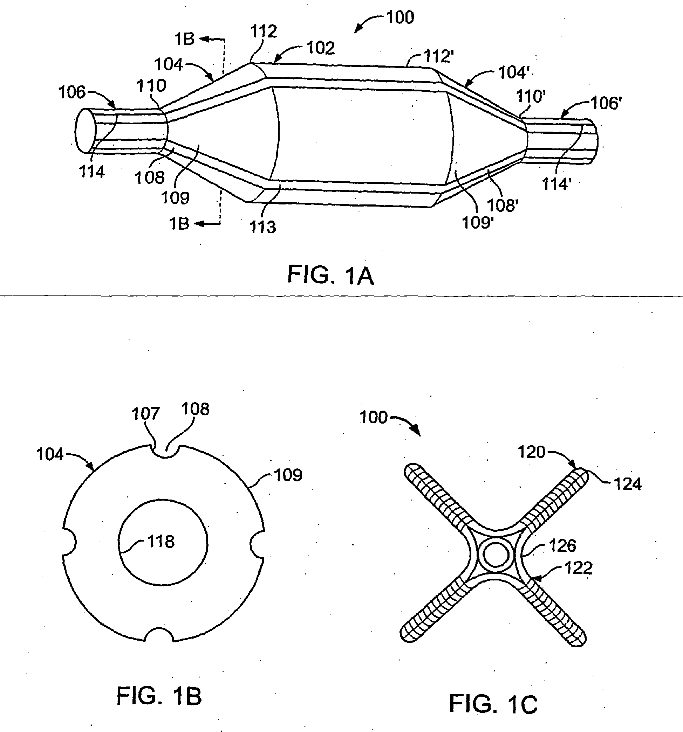

Referring to FIGS. 1A and 1B, a medical balloon 100 includes a plurality of outer recessed regions (as shown, channels 108). As shown, channels 108 extend along the entire length of balloon 100: along a proximal waist portion 106, to along a proximal cone portion 104, to along a body portion 102, to along a distal cone portion 104', and to along a distal waist portion 106'. Along the circumference of balloon 100, outer channels 108 can be equally spaced on the outer surface 109 of the balloon. Channels 108, which can be formed by laser ablation, are capable of enhancing folding of balloon 100 as well as controlling rupture characteristics of the balloon.

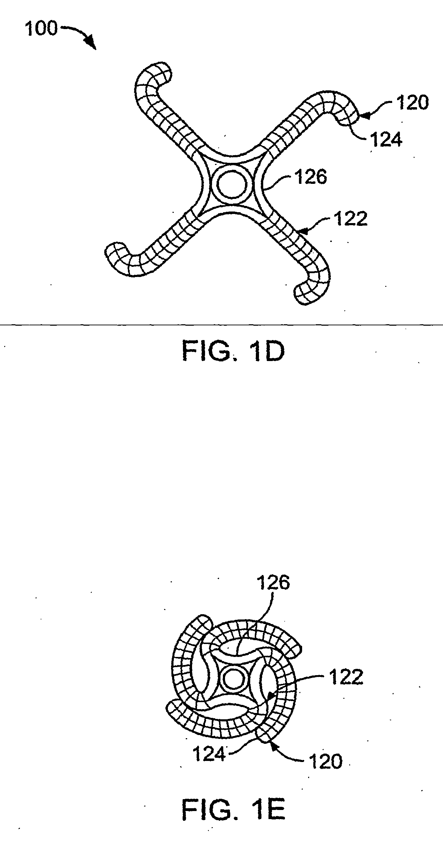

FIGS. 1C, 1D, and 1E show balloon 100 in various stages of folding having multiple peaks 120 and multiple valleys 122 that extend along the length of the balloon. Outer channels 108 are formed at or near the bases 126 of valleys 126. During manufacture of balloon 100, channels 108 serve as preferential folding portions and help to form valleys 122 and peaks 120 so that the balloon can be folded into a preset, compacted shape for delivery into the body. During use, such as when balloon 100 is deflated so that it can be withdrawn from the body, channels 108 again serve as preferential folding portions (e.g., crease lines) that facilitate re-creation of valleys 122 and peaks 120, and re-folding of the balloon into the preset, compacted shape. As a result, the balloon can be quickly, reliably, and easily withdrawn from the patient. Furthermore, as described below, in some embodiments, the depths and/or widths of channels 108 can be formed to enhance the flexibility of balloon and/or to help the balloon assume a small profile when folded.

Moreover, since channels 108 are thinner than other portions of balloon 100, the channels can facilitate a predetermined mode of rupture (such as longitudinal rupture). Under some circumstances, such as when a balloon is unintentionally inflated over its specified limit, the balloon can tear and burst. When the balloon tears transversely to the longitudinal axis of the balloon, the portions of the balloon, particularly the distal portion, attached to the catheter can open up as the balloon is extracted from the body and create a parachute that traps fluids in the body vessel and blocks fluid flow. As a result, it can be difficult to withdraw the balloon catheter from the body. In comparison, when the balloon tears substantially along the longitudinal axis of the balloon, the portions of the balloon attached to the catheter are less likely to trap and to block fluids, thereby allowing the balloon catheter to be easily withdrawn. By forming the relatively thin channel(s) 108 such that they extend generally longitudinally along balloon 100, the balloon is more likely to rupture longitudinally along the channels than to rupture transversely about the balloon.

As described below, various embodiments and combinations of channels 108can be formed on a balloon. Because proximal cone portion 104 and distal cone portion 104' are similar to one another in some embodiments, only the proximal cone portions is described. Similarly, because proximal waist portion 106 and distal waist portion 106' are similar to one another in some embodiments, only the proximal waist portion is described. However, in other embodiments, proximal cone portion 104 and distal cone portion 104' and/or proximal waist portion 106 and distal waist portion 106' are dissimilar to one another.

Still referring to FIGS. 1A-1E, the plurality of channels 108 can be equally spaced apart about the circumference of balloon 100 (as shown), or the channels can be unequally spaced apart about the circumference. Equally spaced apart channels 108 can create a uniform profile when balloon is folded. Unequally spaced channels, for example, such that some circumferential regions include more or fewer channels than other regions, can allow preselected regions to fold sequentially prior to other regions. A balloon can have two or more channels 108, for example, three, four, five, six, seven, eight or more. In some embodiments, a balloon includes only one channel 108.

Channels 108 can extend the entire length of a balloon (e.g., as shown in FIG. 1A), or the channels can extend only along one or more selected portions of the balloon. For example, channel 108 need not extend from one end of a cone portion to another end of the cone portion, but the channel can extend in one or more regions (e.g., the central regions) between the ends. In some embodiments, it may be less desirable for the balloon to rupture in certain regions of the balloon (such as near its proximal end) than in other regions (such as near its distal end). If the balloon ruptures in the proximal end, it can be difficult to remove the balloon from the patient because of a potentially large parachute effect as described above. Accordingly, in some embodiments, the distal regions of a balloon can include one or more channels, while the proximal regions do not include any channels or fewer channels. Generally, one region (such as the distal region) can include more channels than another region, wider channels, and/or deeper channels to facilitate a predetermined folding and/or rupture characteristic.

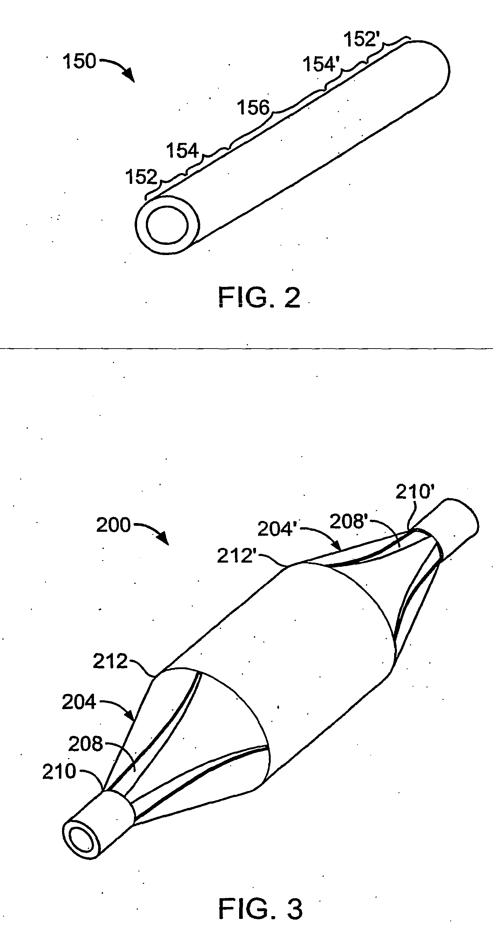

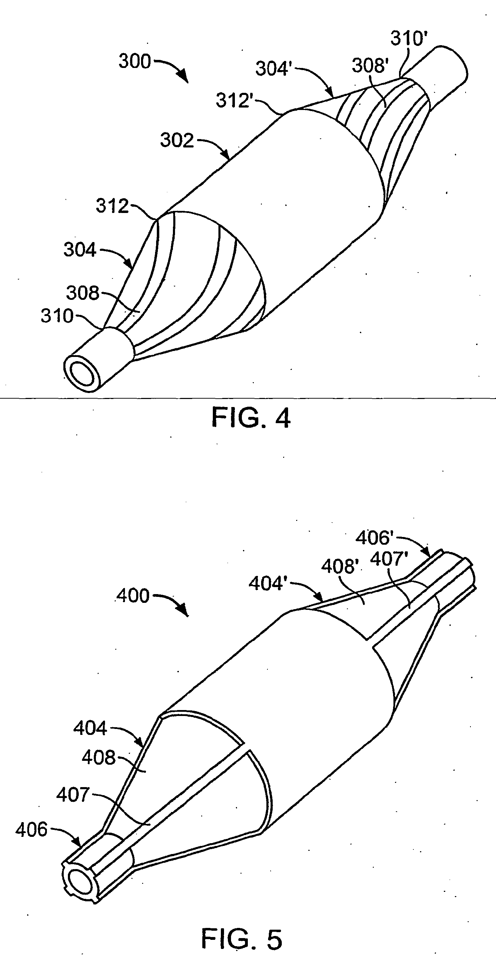

In some manufacturing processes, a balloon is formed by molding (e.g., blow molding) a tube to form the balloon. Molding a balloon can form relatively thick-walled cone portions, which can reduce the flexibility and trackability of the balloon. For example, during molding, the body portion of the balloon can be stretched diametrically by at least a factor of six. As a result, the balloon wall in the body portion can be relatively thin because of the relatively large amount of stretching. However, portions of the balloon other than the body portion may stretch relatively little. The ends of the balloon, for example, may remain approximately the same diameter as the tube and may be stretched by approximately a factor of two. Consequently, the portions of the balloon other than the body portion can remain relatively thick and be inflexible, which can limit the folding of the balloon into a compact profile. By forming the channels on the cone portions and/or the waist portions, folding of the balloon is facilitated in these relatively thick portions, which can help decrease the profile of the balloon and allow the balloon to access narrow bodily vessels during use. For example, referring to FIGS. 3 and 4 which do not form part of the invention, channels 208 an 308 can extend along the cone portions of the balloon only. In some embodiments, referring to FIG. 5, channels 408 can extend along the waist portions and the cone portion to enhance the flexibility and foldability of these portions. The relatively thin body portion can be modified with other features, for example, for endoprosthesis retention, as described below.

Channels 108 can extend linearly or non-linearly along a balloon. For example, channels 108 can extend in serpentine pattern, a zig-zag pattern, a helical pattern, or a curved pattern. Referring to FIG. 4, a balloon 300 includes curved channels 308 that extend from a first end 310 of a cone portion 304 to a second end 312 of the cone portion 304. As a result, balloon 300 tends to collapse or deflate in a counter clockwise direction (as viewed toward body portion 302) to help the balloon to assume a lower profile when folded. Curved channels 308 can also be arranged in an opposite configuration to cause balloon 300 to fold in a clockwise motion (as viewed toward body portion 302). In some embodiments, one of the cone portions 304, 304' can include channels configured to cause folding in a clockwise motion and the other of the cone portions 304, 304' can include channels configured to cause folding in a counterclockwise motion.

Along predetermined paths of the balloon 100, channels 108 can extend continuously or interruptedly. For example, referring again to FIG. 1A, balloon 100 includes four paths, each of which is defined by one channel 108 extending continuously along the path. In other embodiments, each path can be defined by a plurality of channels 108, e.g., aligned end-to-end and spaced equally or unequally, and regularly or irregularly. The paths can extend linearly or non-linearly, as described above. In certain embodiments, each channel can be formed of multiple recesses that are closely spaced apart from one another. The recesses, for example, can be aligned with one another or staggered along the balloon surface. The regions of the balloon that include the spaced apart recesses can have properties that differ from other regions of the balloon that include no recesses. Consequently, such regions can enhance the ease with which the balloon can inflate, deflate, and/or fold.

Channels 108 can be formed to have any of a variety of cross-sectional shapes. Examples of shapes include V-shaped, polygonal (e.g., rectangular or square), oval, and semi-circular. Within a balloon, the cross-sectional shapes of channels 108 can be all the same, all different, or any preselected combination of shapes.

The depth of channel 108 can be substantially constant along its length, or the depth can vary along the length of the channel. The depth can increase or decrease (linearly or nonlinearly) as the channel extends along the cone regions from the waist portion to the body portion. Varying the depth (e.g., increasing the depth or reducing the thickness) of a channel can enhance the flexibility and foldability of the portion of the balloon where the channel is located because the channel has less material than other portions. In some embodiments, the depth of channel 108 can be at least about 1% of the balloon wall thickness, and/or about 95% or less of the balloon wall thickness (e.g., about 75% or less, about 25% or less, from about 5% to about 75%). In certain embodiments, the depth of channel 108 can be nano-sized (less than about 1000 nm). For example, the depth of channel 108 can be up to about 50 microns (e.g., from about 0.5 micron to about 25 microns). In some embodiments, the depth of channel 108 can be less than about 750 mn (e.g., less than about 500 nm). In some embodiments, channels 108 at the distal region of a balloon, or portions of channels at the distal regions, are relatively deeper than other channels or other portions of the channels to facilitate collapse or deflation of the distal region prior to other regions, e.g., the proximal region of the balloon. By causing the distal region of the balloon to collapse first, for example, the fluid inside the balloon can encounter less resistance as it is extracted from the balloon. In particular, the collapsing distal end helps to force the fluid toward the proximal end, thereby assisting the deflation process. In comparison, if the proximal region collapses prior to the distal region, then the fluid traveling out of the balloon from the distal end can encounter the collapsed region of the balloon at the proximal end, thus making it more difficult to remove the fluid from the balloon. In some embodiments, the depth of a channel can be constant along a predetermined portion, and variable along another portion. A balloon can include channels of constant depths, different depths, or any combination of depths.

Similar to the depth, the width of channel 108 can be substantially constant along its length, or the width can vary along the length of the channel (as shown, for example, in FIGS. 3 and 5). The width can increase or decrease (linearly or nonlinearly) as the channel extends along the cone regions from the waist portion to the body portion. As with varying the depth, varying the width (e.g., increasing the width) of a channel can enhance the flexibility and foldability of the portion of the balloon where the channel is located because the channel has less material than other portions. In certain embodiments, the width of channel 108 can be up to about 1500 microns (e.g., from about one micron to about 1000 microns). In some embodiments, the width of channel 108 can be nano-sized (less than about 1000 nm). For example, the width can be less than about 750 nm (e.g., less than about 500 nm). In some embodiments, channels 108 at the distal region of a balloon, or portions of channels at the distal regions, are relatively wider than other channels or other portions of the channels to facilitate collapse or deflation of the distal region prior to other regions, e.g., the proximal region of the balloon, as described above. The channels in the cone portions can be wide to provide flexibility similar to that of the body portion (e.g., to compensate for the difference in thickness).

Other examples of channels having variable widths are also possible. For example, referring to FIG. 5 which refers to an example not part of the invention, a balloon 400 includes channels or variable widths in the form of recessed panels 408 on its cone portion 404 and its waist portion 406. The portions between panels 408 are relatively raised to form raised ribs 407. Recessed panels 408 enhance the foldability of balloon 400 and reduce the profile of the balloon when folded, and ribs 407 provide waist portion 406 and cone portion 404 with good strength and support. Similar to the channels described above, recessed panels 408 can be substantially centered about bases of valleys created by the balloon when deflated. By positioning recessed panels 408 about the bases of the valleys, the panels assist in the formation of the valleys upon deflation of balloon 400 because the panels include less material and are relatively more flexible.

As shown, ribs 407 extend in a substantially linear fashion along the length of waist portion 406 and cone portion 404, but in other embodiments, the ribs can be formed in any of various other shapes and sizes, such as curved, zig-zag shaped, and/or serpentine shaped. Furthermore, ribs 407 need not extend across both cone portion 404 and waist portion 406, but can, for example, be located on the cone portion only. In some embodiments, the cone portion includes ribs, while the waist portion remains unaltered (e.g., not laser ablated) from the starting tube and, therefore, includes no ribs.

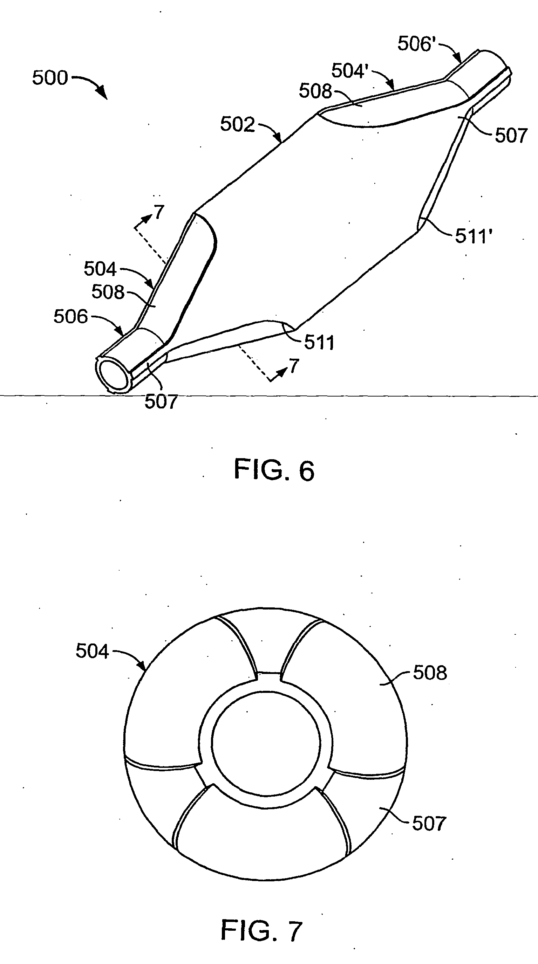

In other examples not forming part of the invention referring to FIGS. 6 and 7, a balloon 500 includes a waist portion 506 and a cone portion 504 having curved recessed panels 508 and relatively raised ribs 507. The end region of the recessed panels 508 nearest body portion 502 of the balloon 500 is defined by a curved wall 511. Due to the shape of recessed panels 508, ribs 507 become wider as they extend near body portion 502, which enhances the strength of balloon 500 at the relatively thin body portion. As a result, balloon 500 can remain capable of withstanding the pressures and stresses imposed during inflation.

The medical balloon described above can be formed by blow molding a polymer tube to form a balloon, and subsequently laser ablating the balloon to form the desired features, such as channels.

The tube can include one or more biocompatible polymers suitable for use in a medical device, for example, thermoplastics and thermosets. Examples of thermoplastics include polyolefins, polyamides, such as nylon 12, nylon 11, nylon 6/12, nylon 6, and nylon 66, polyesters, polyethers, polyurethanes, polyureas, polyvinyls, polyacrylics, fluoropolymers, copolymers and block copolymers thereof, such as block copolymers of polyether and polyamide, e.g., Pebax®; and mixtures thereof. Examples of thermosets include elastomers such as EPDM, epichlorohydrin, nitrile butadiene elastomers, silicones, etc. Thermosets, such as expoxies and isocyanates, can also be used. Biocompatible thermosets may also be used, and these include, for example, biodegradable polycaprolactone, poly(dimethylsiloxane) containing polyurethanes and ureas, and polysiloxanes. Other materials are disclosed in U.S.S.N. 10/645,014, entitled "Multilayer Medical Device" and filed on August 21, 2003.

The tube can include one layer or multiple layers (e.g., by coextrusion) of material. Having a multitude of thin layers can distribute stresses and defects, such as cracks or punctures, so that they are less likely to propagate through the wall of the balloon to the point of causing a failure. Different layers formed of different hardness can assist in distributing stress and retard defect propagation, while providing high burst strength and low distention. The layers can be formed to be thicker than the typical size of defects, e.g., gas bubbles formed in the extrusion or foreign particles.

The tube can be prepared, for example, by an extrusion process. For multilayered tubes, in some embodiments, the extrusion process involves the use of an extrusion apparatus (e.g., a crosshead, such as a compact crosshead) having a series of discs. Examples of extrusion apparatuses, including some illustrative operating conditions, such as zone heating temperatures, polymer concentrations, feed rate, and line speed, are described in commonly assigned U.S.S.N. 09/798,749, entitled "Multilayer Medical Device" and filed on March 2, 2001, and U.S.S.N. 10/645,014, also entitled "Multilayer Medical Device" and filed on August 21, 2003. An exemplary system for controlling the feed rate or flow of polymers, including melt pumps, and systems and methods for controlling the pumps, is also described in WO 01/32398, entitled "Method and Apparatus for Extruding Catheter Tubing". Other methods include using servo-controlled valves, as described in Burlis et al., U.S. Patent No. 3,752,617.

To form a balloon, the formed (e.g., co-extruded) tube can be blow molded. In some embodiments, the tube is placed (e.g., centered) in a preheated balloon mold, and air is introduced into the tube to maintain the patency of the tube lumen. After soaking at a predetermined temperature and time, the tube is stretched for a predetermined distance at a predetermined time, rate, and temperature. The pressure inside the tube is then sufficiently increased to radially expand the tube inside the mold to form the balloon. Methods of forming a balloon from the tube are described, for example, in commonly-assigned U.S.S.N. 10/263,225, filed October 2, 2002, and entitled "Medical Balloon"; U.S.S.N. 10/645,055, filed August 21, 2003, and entitled "Medical Balloon' ; Anderson, U.S. 6,120,364; Wang, U.S. 5,714,110; and Noddin, U.S. 4,963,313. In embodiments, the formed balloon is heat treated, for example, to enhance folding memory, and/or folded into a predetermined profile.

The recessed portions (e.g., channels 108) and other features described herein can be created by laser ablating predetermined areas of the balloon and/or the parison from which the balloon is formed. In some embodiments, laser ablation is performed using a ultraviolet (UV) light laser. The depth of ablation can be controlled by adjusting, for example, the wavelength of the incident light and/or the energy fluence (J/cm²). The UV light, for example, can be applied in pulses. In some embodiments, UV light having a wavelength of about 157 nm to about 450 nm (e.g., about 157 nm to about 350 nm, about 157 nm, about 193 nm, about 248 nm, about 450 nm) can be used. The smoothness of an ablated surface can be a function of the wavelength of the UV light, e.g., the smoothness of the ablated surface can increase with the wavelength. Laser ablation using light with a relatively short wavelength (e.g., about 157 nm) can, therefore, be used to create a high quality surface that has few cracks (e.g., relative to grinding) and is resistant to failure. The energy fluence can range from about 0.05 J/cm² to about 5.0 J/cm². For example, for a parison or a balloon formed of polyamide, UV light having a wavelength of about 193 nm and an energy fluence of about 0.06 J/cm² to about 1.5 J/cm² can remove about 0.46 micrometers or more of material per pulse of about 20 nanoseconds.

In some embodiments, the tube or parison 150 (shown in FIG. 2) can be ablated prior to forming of the balloon in order to achieve an effect similar to those discussed above. For example, referring to FIG. 2, areas of the mid region 154 of parison 150 can be ablated to form channels 108 in cone portion 104 of the balloon 100. Similarly, areas of the central region 156 and/or the end region 152 of the parison 150 can be ablated to form channels on the balloon's body portion 102 and/or waist portion 106, respectively.

### Endoprosthesis Retention

As indicated above, in addition to forming recessed regions on a balloon, laser ablation can be used to form features to help retain an endoprosthesis (such as a stent, a stent-graft, or a covered stent) on the balloon. For example, the surface of the balloon can be roughened to increase friction between the balloon and the endoprosthesis; the surface of the balloon can include one or more raised features (e.g., rings and/or protrusions); and/or a soft layer and/or an adhesive layer can be applied to the surface of the balloon.

In some embodiments, the outer surface of a balloon (such as on the body portion) is roughened to reduce movement of an endoprosthesis carried by the balloon. For example, in some embodiments the wall thickness of the balloon can be up to about 0.5 mm (e.g., from about 0.0076 mm to about 0.33 mm). , and sub-micron size features can be created on the surface of the balloon by contacting UV laser light to the balloon. The sub-micron size features extending from the surface of the balloon, for example, can be spiked, sinusoidal, skewed, and/or pillar shaped. The sub-micron size features can help retain an endoprosthesis on the balloon, without substantially compromising the structure and strength of the balloon. The energy can be directed to closely spaced apart regions of the balloon in order to create surface regions of different roughness. Alternatively or additionally, a laser with a nonhomogeneous energy distribution can be directed to the balloon in order to create surface regions of differing roughness. In some embodiments, the roughened surface (e.g., of the body portion) of the balloon can have an average roughness (Rₐ) of about one micrometer or more (e.g., about one micrometer to about 10 micrometers), as measured with a pertometer. As an example, a XeCl excimer at 800 mJ/cm² can provide a roughness (Rₐ) of about one micrometer on the surface of PET with a single pulse. As another example, a nonhomogenous laser beam having an energy fluence ranging from below the threshold level to about 800 mJ/cm² can provide a roughness (Rₐ) of about 0.5 micrometer on the surface on the surface of PET with a single pulse. It should be appreciated that the roughness can be increased by applying multiple pulses of energy to the material being ablated. It should also be appreciated that the roughness can be increased by increasing the energy fluence and can be decreased by decreasing the energy fluence.

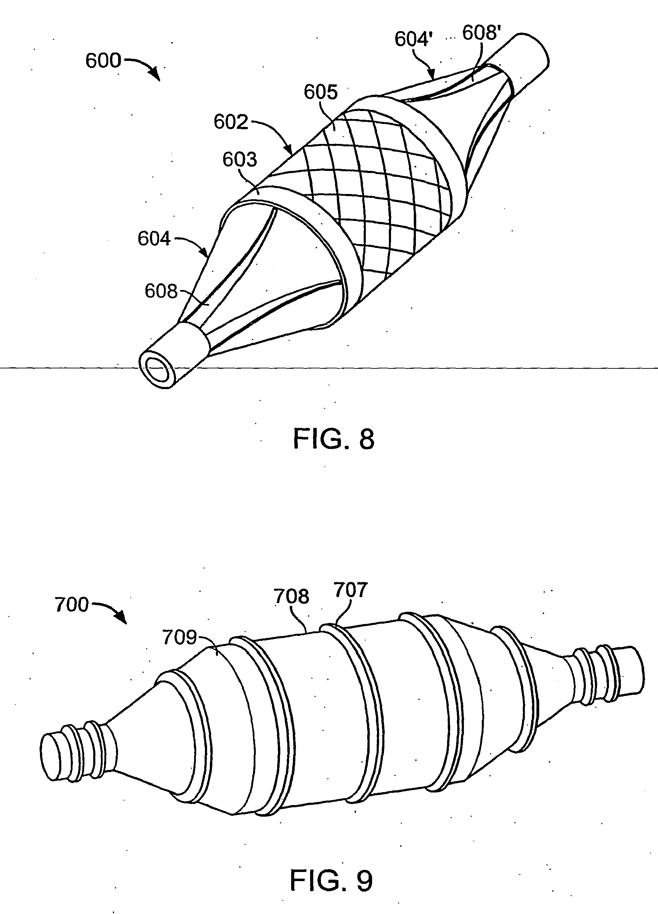

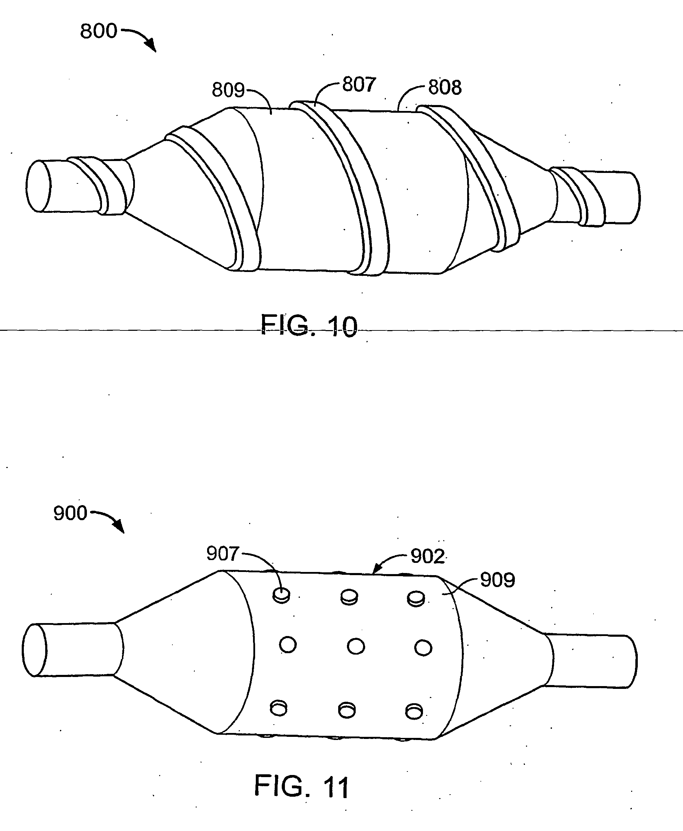

As an alternative to or in addition to roughening the surface of the balloon, one or more raised features can be created to enhances retention of the endoprosthesis. Referring to FIG. 9 which refers to an example not part of the invention, a balloon 700 includes a plurality of rings 707 that are raised relative to other portions 708 of the balloon. When balloon 700 is carrying an endoprosthesis, rings 707 help to prevent the endoprosthesis from moving (e.g., sliding) longitudinally and/or circumferentially along the surface of the balloon, such as during delivery of the endoprosthesis. As shown, rings 700 extend substantially perpendicular to the longitudinal axis of balloon 700. In other embodiments, one or more relatively raised can extend in a different manner. For example, referring to FIG. 10 which refers to an example not part of the invention, a balloon 800 includes a raised feature (as shown, a coil 807) that extends helically about an outer surface 809 of the balloon 800.

A number of embodiments of rings 707 and coil 807 can be formed. In some embodiments, rings 707 have a height of about 10 percent or more (e.g., about 10 percent to about 100 percent, about 10 to about 50 percent) of the thickness of a stent to be positioned thereon. For example, rings 707 and coil 807 can have a height of about 10 micrometers to about 100 micrometers (e.g., about 10 micrometers or more, about 20 micrometers or more, about 30 micrometers or more, about 40 micrometers or more, about 50 micrometers or more, about 60 micrometers or more, about 70 micrometers or more, about 80 micrometers or more, about 90 micrometers or more, about 100 micrometers or less, about 90 micrometers or less, about 80 micrometers or less, about 70 micrometers or less, about 60 micrometers or less, about 50 micrometers or less, about 40 micrometers or less, about 30 micrometers or less, about 20 micrometers or less). A balloon can have rings 707 of different heights and/or widths, or the heights and/or widths can be the same.

Adjacent rings 707 can be spaced apart along balloon 700 by a substantially uniform distance, or one or more of the pairs of adjacent rings can be separated by unequal distances. For example, rings near the center of the balloon can be spaced apart by relatively small distances while rings near the end regions of the balloon can be separated by relatively larger distances. Rings 707 or coil 807 can extend along the outer surface of the balloon at any angle greater than zero degrees to less than 180 degrees, relative to the longitudinal axis of the balloon. Rings 707 or coil 807 can extend continuously or interruptedly along the surface of the balloon. Rings 707 and coil 807 need not extend substantially linearly around the balloon as shown but can extend, for example, in a curved, zig-zag, or serpentine fashion around balloon 700.

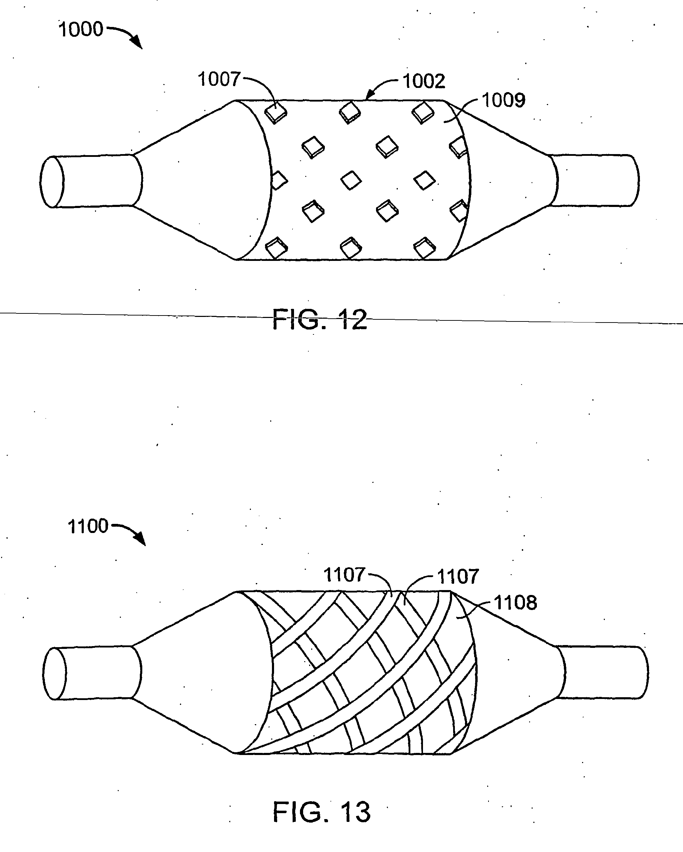

Still other embodiments of raised features can be formed to enhance retention of an endoprosthesis to a balloon. Referring to FIG. 11 which shows an example not part of the invention, a balloon 900 includes an array of cylindrical protrusions 907 extending from its body portion 902. Protrusions 907 can be arranged to mate or to align with apertures of an endoprosthesis to be carried by balloon 900. For example, when a stent is positioned on body portion 902 of balloon 900, protrusions 907 extend through the apertures of the stent to securely retain the stent in a desired position. Balloons can also include protrusions of various other shapes and sizes. As shown in FIG. 12, which shows an example not part of the invention, a balloon 1000 includes multiple rectangularly shaped protrusions 1007 extending from an outer surface 1009 of a body portion 1002 of balloon 1000. Protrusions 1007 can also be arranged to mate with apertures of a stent that is carried by balloon 1000. FIG. 13 shows an example not part of the invention where a balloon 1100 includes raised features or protrusions 1107 that are interconnected, as shown, in a lattice configuration. The raised features can have dimensions similar to those described above for rings 707 and coil 807. The height and width of protrusions 907, 1007, 1107 can be similar to the height and width of rings 707 discussed above.

The raised portions (e.g., rings 707, coil 807, and protrusions 907, 1007, 1107 described herein) can be formed by laser ablating the balloon or the tube from which the balloon is formed. For example, to create the lattice configuration of protrusions 1107, multiple rectangular areas of the balloon are ablated to form a plurality of rectangular recessed regions 1108. The ablated regions 1108 are arranged such that the remaining unablated regions form the lattice configuration of interconnected protrusions 1107. Similarly, rings 707, coil 807, and the other protrusions can be formed by laser ablating predetermined areas of the parison or the balloon that do not define these features.

The raised portions can include a composition that is the same as the composition of the relatively recessed (e.g., unablated) areas, or the compositions of the raised portions and the recessed areas can be different. By forming the raised portions to include a relatively softer material (e.g., Pebax 25D), for example, an endoprosthesis can better settle into the raised portions to more securely retain the endoprosthesis. A relatively soft raised portion can be formed by coextruding a tube having an inner layer and a relatively soft outer layer. The tube can be laser ablated to form the raised portions, and/or the tube can be formed into a balloon, and the balloon is subsequently laser ablated to form the raised portions. A raised portion can include one layer (e.g., the outer layer of a coextruded balloon); in some embodiments, a raised portion can include multiple layers, such as a soft outer layer and a hard, underlying layer. In some embodiments, one or more layers (such as the outer layer, e.g., including SIBS) can include a drug (such as paclitaxel).

While the balloons described above have raised portions in certain areas of the balloons, in other embodiments, the raised portions can be in any combination of the areas. For example, rings 707 and/or coil 807 can be formed only on the body portion, only on one or both waist portions, only on one or both cone portions, on in any combination of these areas. Similarly, the protrusions shown in FIGS. 11, 12, and 13 can be formed only on the body portion, only on one or both waist portions, only on one or both cone portions, on in any combination of these areas.

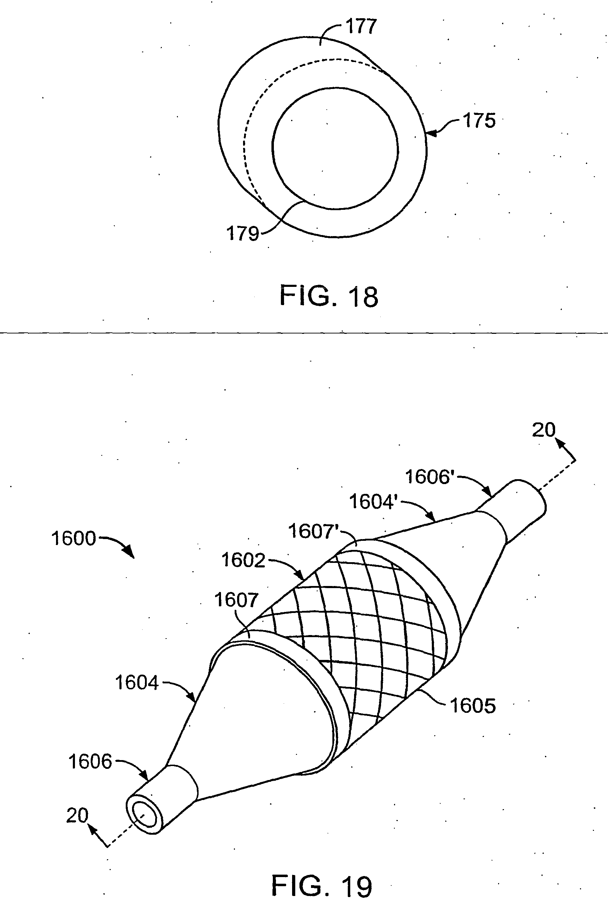

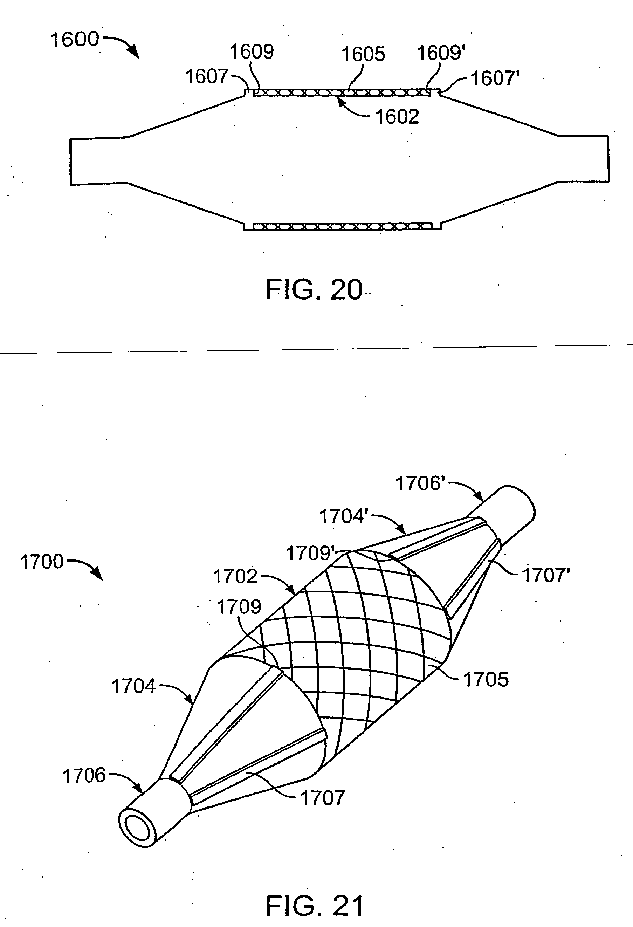

In some embodiments, stop features are included at opposite ends of a balloon to help prevent an endprosthesis from axially sliding about the balloon. The stop features can also help prevent the distal and/or proximal edges of the endoprosthesis from snagging a wall of a bodily vessel as the endoprosthesis is being fed into and/or retracted from the bodily vessel of a subject. Referring to FIGS. 19 and 20 which show examples not part of the invention, a balloon 1600 includes rings 1607, 1607' at proximal and distal end regions of a body portion 1602 of balloon 1600. Rings 1607, 1607' extend circumferentially about the proximal and distal ends, respectively, of body portion 1602. Rings 1607, 1607' extend to a height above the surface of body portion 1602, and include surfaces 1609, 1609' facing inward toward body portion 1602. An endoprosthesis 1605 is retained on body portion 1602 (e.g., by an interference fit) between rings 1607, 1607' such that proximal and distal edges of endoprosthesis 1605 abut surfaces 1609, 1609'. Consequently, substantial axial movement of endoprosthesis 1605 about balloon 1600 can be prevented.

Rings 1607, 1607' can have a height sufficient to resist axial movement of endoprosthesis 1605 about balloon 1600. In some embodiments, rings 1607, 1607' have a height substantially equal to the height of endoprosthesis 1605. However, rings 1607, 1607' can have a height greater than or less than the height of endoprosthesis 1605. In some embodiments, for example, rings 1607, 1607' have a height of about 10 percent or more (e.g., 50 percent or more, 100 percent or more, about 10 percent to about 50 percent, about 50 percent to about 100 percent) of the thickness of endoprosthesis 1605. For example, rings 1607, 1607' can have a height of about 10 micrometers to about 100 micrometers (e.g., about 10 micrometers or more, about 20 micrometers or more, about 30 micrometers or more, about 40 micrometers or more, about 50 micrometers or more, about 60 micrometers or more, about 70 micrometers or more, about 80 micrometers or more, about 90 micrometers or more, about 100 micrometers or less, about 90 micrometers or less, about 80 micrometers or less, about 70 micrometers or less, about 60 micrometers or less, about 50 micrometers or less, about 40 micrometers or less, about 30 micrometers or less, about 20 micrometers or less). In some embodiments, endoprosthesis 1605 has a thickness of about 0.08 mm to about 0.20 mm. In certain embodiments, proximal ring 1607 has a height that is unequal to the height of distal ring 1607'. For example, proximal ring 1607 can have a greater height than distal ring 1607'. Consequently, proximal ring 1607 can provide greater resistance to axial movement of endoprosthesis 1605 than distal ring 1607' in some embodiments. Alternatively, proximal ring 1607 can have a height less than the height of distal ring 1607'.

In certain embodiments, edges of rings 1607, 1607' can be rounded or tapered. This can reduce the likelihood of rings 1607, 1607' snagging a wall of the bodily vessel through which balloon 1600 is inserted and retracted. Such snagging of the bodily vessel wall can lead to damage to the bodily vessel wall and/or can cause the endoprosthesis to prematurely deploy.

Balloon 1600 can be formed using methods similar to those described herein. For example, a parison having a wall thickness greater than the desired wall thickness of the balloon to be formed can be blow molded and stretched to form a balloon having relatively thick walls. And then, regions of the balloon can be laser ablated in order to form rings 1607, 1607'. For example, the majority of body portion 1602 can be laser ablated, leaving only proximal and distal portions of body portion 1602 to have their original thickness. The unablated regions can become rings 1607, 1607'. In certain embodiments, the parison used to form balloon 1600 is laser ablated prior to being blow molded. For example, regions of the parison other than those regions corresponding to regions of a balloon where rings 1607, 1607' are desired can be ablated prior to forming the balloon. In some embodiments, cone portions 1604, 1604' and waist portions 1606, 1606' are also laser ablated to reduce the wall thickness in those regions, which can reduce the profile of the balloon in the inflated and deflated states. In certain embodiments, one or more of the laser ablation techniques described herein can also used to enhance the ability of balloon 1600 to deflate in a predetermined manner and/or to reduce (e.g., minimize) the profile of balloon 1600 when deflated and folded.

While the embodiments described above involve forming rings 1607, 1607' from the balloon material, other techniques can be used. In some embodiments, for example, a material, such as a polymer and/or a solgel based material, is applied to those regions of balloon 1600 in which rings are desired. The added material can be attached to the balloon surface using any of various techniques, such as laser bonding, welding, and/or adhesion. In some embodiments, the added material is laser ablated to form rings 1607, 1607'. However, in certain embodiments, laser ablation may not be required. For example, the added material can be applied to the balloon surface in the size and shape of rings 1607, 1607'.

Other types of stop features can also be used to axially retain and enoprosthesis on a balloon and/or to help prevent distal and proximal edges of the endoprosthesis from getting hung up on a wall of a bodily vessel. As shown in FIG. 21, which relates to an example not part of the invention, a balloon 1700 includes multiple ribs 1707, 1707' extending along cone portions 1704, 1704' of balloon 1700. Ribs 1707, 1707' extend to a height above the surface of body portion 1602, and include surfaces 1709,1709' facing inward toward body portion 1702. Surfaces 1709, 1709' abut the proximal and distal edges, respectively, of endoprosthesis 1705 when it is positioned on body portion 1702 of balloon 1700. Thus, ribs 1707, 1707' can help prevent substantial axial movement of endoprosthesis 1705 about balloon 1700. Ribs 1707, 1707' can have a height similar to the height of rings 1607, 1607' discussed above. The heights of ribs 1707, 1707' need not be equal to one another. Ribs 1707, 1707', as shown in FIG. 21, have a substantially uniform width. However, ribs 1707, 1707' can alternatively or additionally be formed to increase or decrease in width along cone portions 1704, 1704'. For example, ribs 1707, 1707' can have a smaller width near waist portions 1706, 1706' and a larger width near body portion 1702. In certain embodiments, the ribs include round or tapered edges, which can help prevent the ribs from becoming snagged on the walls of bodily vessels. In some embodiments, ribs 1707, 1707' are circumferentially spaced apart about cone portions 1704, 1704' in an arrangement that enhances the ability of balloon 1700 to fold, as described in the section above.

Balloon 1700 can be formed using techniques similar to those described herein. For example, ribs 1707, 1707' can be formed by laser ablating the balloon and/or by laser ablating the parison prior to forming the balloon. Alternatively or additionally, other materials can be attached to the balloon surface in order to create ribs 1707, 1707'.

### Drug Eluting Laver

Laser ablation can also be used to form a drug eluting layer on a balloon. Referring to FIG. 8, a balloon 600 includes a cone portion 604 having curved recessed channels 608, and a body portion 602 including a drug-eluting layer 603. As shown, balloon 600 is carrying a stent 605. Drug-eluting layer 603 can include a polymer (for example, a bioerodible polymer), and a drug or a therapeutic agent (such as paclitaxel). In some embodiments, the softness of the material from which drug-eluting layer 603 is formed helps to retain stent 605 on balloon 600 by allowing the stent to securely settle within the soft drug-eluting layer. As shown, drug-eluting layer 603 extends beyond the ends of stent 605, but in other embodiments, the ends of the drug-eluting layer can extend to anywhere along the length of balloon 600.

Drug-eluting layer 603 can be formed by laser ablating a multilayered tube and forming a balloon from the tube. Alternatively or additionally, the multilayered balloon can be laser ablated. For example, the multilayered tube can be formed by coextrusion. Subsequently, selected areas of the tube or the balloon (such as the cone portions and the waist portions) can be removed by laser ablation to form layer 603.

During use, the therapeutic agent can be released from drug-eluting layer 603 while stent 605 is implanted within a subject's partially occluded bodily vessel.

### Cutting Balloons

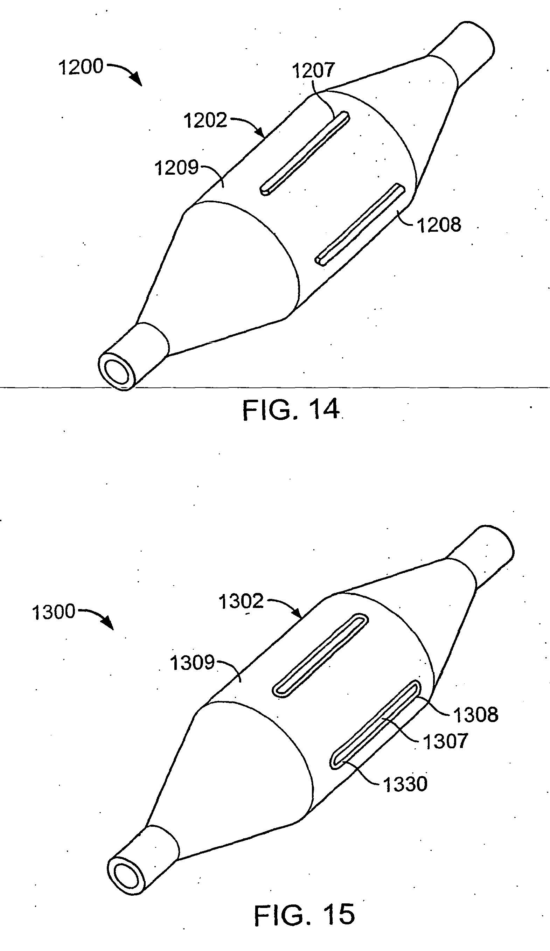

In addition to the applications described above, laser ablation can be used to form cutting elements or blades on balloons. Referring to FIG. 14, a balloon 1200 includes multiple cutting blades 1207 on its body portion 1202. More specifically, cutting blades 1207 can be formed by laser ablation such that they are integrally formed with balloon 1200 and extend from an outer surface 1209 of the balloon. The integrally formed cutting blades are well secured to the balloon, thereby preventing the blades from falling off the balloon. Balloons having cutting elements are described, for example, in U.S.S.N. 10/335,604, filed January 2, 2003.

In some embodiments, cutting blades 1207 can be formed by laser ablating one or more outer layers of a multilayered tube or one or more layers of a multilayered balloon. The tube can be formed by coextrusion, and the balloon can be formed by blow molding the coextruded tube. The outer layers (s) can generally be formed of a material having a hardness sufficient for a particular use. In some embodiments, the material is a biocompatible, hard material. Further examples of materials are those including a liquid crystalline polymer and/or PEEK. Other materials are described in commonly assigned U.S. Publication No. 2002/0165523, filed March 2, 2001.

Cutting blades 1207 can be formed by ablating selected regions of body portion 1202 of the initially formed balloon or parison except those regions where the cutting blades are defined. In other words, one or more layers of material are removed from body portion 1202 to provide an ablated region 1208 from which cutting blades 1207 extend.

During use, balloon 1200 can be inserted into a subject's bodily vessel, and expanded at a desired point within the bodily vessel. Cutting blades 1207 can cut, for example, plaque or a calcified material that is occluding a bodily vessel, thereby facilitating dilation of the vessel.

In other embodiments, laser ablation can be used to help secure a cutting blade (for example, a metal blade) to a balloon. Referring to FIG. 15, for example, a balloon 1300 includes a body portion 1302 having a recessed region 1308, a filler material 1330 fixed within recessed region 1308, and a cutting blade 1307 attached to or disposed on the filler material. In some embodiments, recessed region 1308 extends into one or more cone regions, for example, to facilitate lining cutting blade 1307 on the recessed region. Filler material 1330 can be formed from a material that is less elastic than the material from which balloon 1300 is formed. For example, filler material 1330 can include one or more materials such as HDPE, ceramic, stainless steel, UV cross-linkable polyester, and nanoparticles having at least one dimension less than about 1000 nm in size (e.g., about 750 nm or less, or about 500 nm or less, for example, from about 1 mn to about 100 nm). The inelasticity of filler material 1330 provides a stable substrate for cutting blade 1307 and prevents recessed region 1308 from expanding or contracting during inflation and deflation of balloon 1300. As a result, cutting blade 1307 is prevented from falling off balloon 1300.

Exemplary nanoparticles include, among others, synthetic or natural phyllosilicates including clays and micas (that may optionally be intercalated and/or exfoliated) such as montmorillonite (mmt), hectorites, hydrotalcites, vermiculite, and laponite; monomeric silicates such as polyhedral oligomeric silsequioxanes (POSS) including various functionalized POSS and polymerized POSS; carbon and ceramic nanotubes, nanowires and nanofibers including single and multi walled fullerene nanotubes, silica nanogels, and alumina nanofibers; metal and metal oxide powders including aluminum oxide (Al₂O₃), titanium oxide (TiO₂), tungsten oxide, tantalum oxide, zirconium oxide, gold (Au), silver (Ag), platinum (Pt) and magnetic or paramagnetic powders such as neodinium iron boron, superparamagnectic ferrite oxide (Fe₃O₄) or superparamagnetic maghemite (Fe₂O₃). Other nanoparticles, including their characteristics, are described in U.S. Published Application 20030093107.

Balloon 1300 can be created using laser ablation. For example, after the starting tube is formed into a balloon, one or more recessed regions 1308 can be created by laser ablating selected regions of the balloon's outer surface 1309. After ablating the desired regions, filler material 1330 is deposited into recessed regions 1308. Then, cutting blade 1307 is attached to filler material 1330, for example, with an adhesive. Alternatively or additionally, cutting blade 1307 can be placed within filler material 1330 while the filler material is in a liquid or semi-solid state such that the filler material solidifies or hardens around a lower portion or surface of the cutting blade. As a result, cutting blade 1307 becomes attached to filler material 1330.

Rather than ablating regions of the balloon or in addition to ablating regions of the balloon, regions of the parison or tube from which the balloon is formed can be ablated prior to forming the balloon. In some embodiments, due to the relative thinness of the parison walls at the ablated regions, the pressure applied against the inner surface of the parison during the blow molding process can cause the walls at the ablated region to bulge outwardly. As a result, the balloon may not include a recessed region having a desired depth, width, and/or length. Thus, prior to blow molding the balloon, a removable material, such as a metal insert or a water dissolvable PVA insert, can be inserted into the recessed region of the parison. When the balloon is formed by the blow molding process the removable material can prevent the ablated regions of the parison from expanding outwardly. After the balloon has been formed, the removable material can be removed from the balloon to expose the recessed region having the desired dimensions. The manufacturing method can proceed, as described above, by depositing filler material 1330 into recessed regions 1308 and attaching cutting blade 1307 to the filler material.

In general, any of the embodiments of balloons described herein can be used with one or more cutting elements, for example, to enhance folding (e.g., faster folding) and/or to reduce total balloon profile.

### Tapered Balloon

As discussed above, in some embodiments it is beneficial for the distal region of a balloon to collapse prior to the proximal region. For example, collapse of the distal region prior to the proximal region can help to direct inflation fluid toward the proximal region, which is the region from which the fluid is typically extracted from the balloon. As a result of the distal region collapsing prior to the proximal region, the balloon can deflate relatively fast.

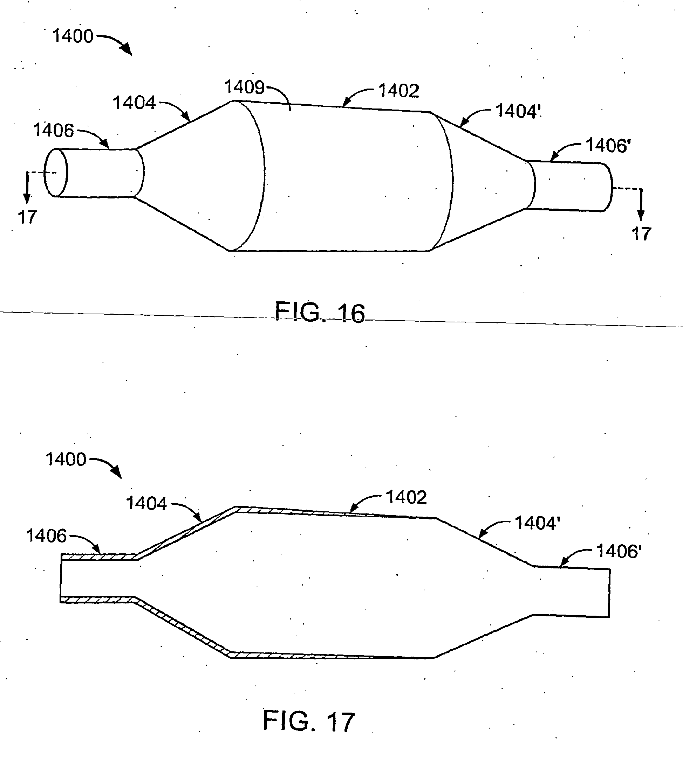

Referring to FIGS. 16 and 17 which show an example not part of the invention, a balloon 1400 is formed such that its wall thickness is greater at a proximal region than at a distal region of the balloon to promote collapse of the distal region prior to the proximal region. As shown, the walls of a distal cone portion 1404' and a distal waist portion 1406' are substantially uniform in thickness. Similarly, the walls of a proximal cone portion 1404 and a proximal waist portion 1406 are substantially uniform in thickness. The walls of a body portion 1402 increase in thickness from the approximate thickness of the proximal cone portion 1404 and proximal waist portions 1406 at the proximal end of the body 1402 to the approximate thickness of the distal cone portion 1404' and the distal waist portion 1406 at the distal end of the body 1402. In other embodiments, the wall thickness of the waist and cone portions may differ. For example, the balloon can have a substantially uniform thickness apart from one or both of the cone portions, which can have thinner walls than the remainder of the balloon.

Tapered balloon 1400 can be made using laser ablation. For example, a balloon having a relatively uniform wall thickness can be ablated with a UV laser so that increasingly more material is removed as progression is made from the proximal end of the balloon to the distal end of the balloon. Alternatively or additionally, the parison from which balloon 1400 is formed can be ablated as described above such that the wall thickness of the parison decreases from its proximal region to its distal region. When the parison is blow molded to form the balloon, the resulting balloon has a wall thickness that decreases from its proximal region to its distal region.

In some embodiments, tapered balloon 1400 can include multiple layers. For example, an outer layer can be coextruded, and the outer layer (of the tube and/or the balloon) can be ablated in the manner described above such that the outer layer has a thickness that decreases from the proximal region to the distal region of the balloon. In some embodiments, the outer layer is formed of a relatively softer material than underlying layer(s) of the balloon to enhance the balloon's stent-retention ability.

### Parison Ablation

Laser ablation can be used on parisons to produce beneficial features other than those features described above. For example, by ablating the parison prior to forming the balloon, a smooth surface is created on the balloon surface, rather than the relatively rough surface that can be created by blow molding a balloon without the use of laser ablation. In some embodiments, substantially the entire surface of the parison is subjected to laser ablation in order to form a balloon with a smooth surface over its entirety. In some embodiments, however, only a select region of the parison is subjected to laser ablation.

Parisons can be also be ablated to make it more regular. In some cases, a parison may have a non-circular cross-section, which can result in a non-regular or asymmetric balloon. As shown in FIG. 18, for example, a parison 175 includes a somewhat elliptical cross-section. Using laser ablation, an outer region 177 of the irregular parison can be laser ablated to form a substantially circular parison 179. For example, the parison can be placed on a computer controlled mandrel, and selected portions (as shown, region 177) can be controllably ablated.

The features described above can be used in any combination. For example, a balloon (such as a cutting balloon) can include features that enhance preferential folding and/or enhanced endoprosthesis retention. A balloon can include multiple endoprosthesis retention features. A tapered balloon can include features that enhance preferential folding and/or enhanced endoprosthesis retention. A balloon catheter can include any of the balloon features described herein, such as integrally formed cutting blades.

Laser ablation can also be performed on other components of a balloon catheter. For example, a balloon catheter may include an inner component (e.g., a tube) that defines a guidewire lumen, and the inner component can be constructed initially of three layers. The three layers may include an inner layer (e.g., including polytetrafluoroethylene), a middle layer (e.g., including a metal braid), and an outer layer (e.g., including high density polyethylene). Laser ablation can be used to selectively remove the outer layer and to expose the middle layer. In some embodiments, laser ablation can further be used to form openings extending through the middle and inner layers, for example, for injection of dyes. Balloon catheters are described, for example, in US-2004-OI31808-A1.

## Claims

1. A method comprising:
providing a medical balloon (100; 200; 300; 400; 500; 600; 1200; 1300) comprising a first cone portion and a body portion (102; 202; 302; 402; 502;602; 1202; 1302); and
removing material from an outer surface of the body portion of the balloon to form first regions and a second regions, the first regions being recessed relative to the second regions,
wherein the first regions form channels (108; 208; 308; 408; 508; 608; 1208; 1308) which extend generally longitudinally along balloon to serve as preferential folding portions.

2. The method of claim 1 , wherein removing material from the balloon comprises laser ablating the balloon.

3. The method of claim 2, wherein the balloon is laser ablated at a wavelength of about 157 nm to about 450 nm.

4. The method of claim 3, wherein the wavelength is about 248 nm.

5. The method of claim 3, wherein the wavelength is about 193 nm.

6. The method of claim 1, further comprising securing a cutting blade within the recessed area.

7. The method of claim 6, further comprising introducing a filler material into the recessed areas.

8. The method of claim 7, wherein the filler material comprises one or more materials selected from the group consisting of HDPE, ceramic, stainless steel, UV cross- linkable polyester, and a nanoparticles.

9. The method of claim 1, further comprising applying a second material to the outer surface of the balloon.

10. The method of claim 9, wherein the second material is harder than the material of the body portion.

11. The method of claim 10, wherein the method comprises ablating regions of the second material to form cutting elements extending from the outer surface of the body portion.

12. The method of claim 9, wherein the second material is softer than the material of the body portion.

13. The method of claim 12, wherein the method comprises removing at least a portion of the second material.

14. The method of claim 13, wherein, after removing the portion of the second material, the second material tapers in thickness from a first end area to a second end area of the body portion.

15. The method of one of the preceding claims, wherein the second regions are longitudinally positioned along the body portion in regular spaced intervals.

16. The method of claim 1, wherein the outer surface of the body portion of the balloon has an average roughness of about 1 micrometer or more.

17. A medical device having a balloon (100; 200; 300; 400; 500; 600; 1200; 1300) comprising a first cone portion and a body portion (102; 202; 302; 402; 502; 602; 1202; 1302);
wherein the body portion of the balloon has first regions and second regions, the first regions being recessed relative to the second regions, wherein the first regions form channels (108; 208; 308; 408; 508; 608; 1208; 1308) reducing the thickness of the balloon, said channels extending generally longitudinally along the balloon to serve as preferential folding portions.

18. The medical device of claim 17, further comprising a filler material in the recessed areas.

19. The medical device of claim 18, wherein the filler material comprises one or more materials selected from the group consisting of HDPE, ceramic, stainless steel, UV cross-linkable polyester, and a nanoparticles.

20. The medical device of claim 17, further comprising a second material on the outer surface of the balloon.

21. The medical device of claim 20, wherein the second material is harder than the material of the body portion.

22. The medical device of claim 21, wherein the second material is softer than the material of the body portion.

23. The medical device of one of claims 17 to 22, wherein the second regions are longitudinally positioned along the body portion in regular spaced intervals.

24. The medical device of claim 17, wherein the outer surface of the body portion of the balloon has an average roughness of about 1 micrometer or more.

25. The medical device of one of the preceding claims, wherein the channels (108; 208; 308; 408; 508; 608; 1208; 1308) are formed to facilitate a predetermined mode of rupture.

## Patentansprüche

1. Verfahren, umfassend:
Bereitstellen eines medizinischen Ballons (100; 200; 300; 400; 500; 600; 1200; 1300), der einen ersten Kegel-Teilbereich und einen Körper-Teilbereich (102; 202; 302; 402; 502; 602; 1202; 1302) umfasst; und Entfernen von Material von einer äußeren Oberfläche des Körper-Teilbereichs des Ballons, um erste Bereiche und zweite Bereiche zu bilden, wobei die ersten Bereiche vertieft sind relativ zu den zweiten Bereichen,
wobei die ersten Bereiche Kanäle (108; 208; 308; 408; 508; 608; 1208; 1308) bilden, die sich im Wesentlichen longitudinal entlang des Ballons ausdehnen, um als bevorzugte Falt-Teilbereiche zu dienen.

2. Verfahren gemäß Anspruch 1, wobei Entfernen von Material von dem Ballon Laserablation des Ballons umfasst.

3. Verfahren nach Anspruch 2, wobei der Ballon bei einer Wellenlänge von ungefähr 157 nm bis ungefähr 450 nm laserablatiert wird.

4. Verfahren gemäß Anspruch 3, wobei die Wellenlänge ungefähr 248 nm beträgt.

5. Verfahren nach Anspruch 3, wobei die Wellenlänge ungefähr 193 nm beträgt.

6. Verfahren nach Anspruch 1, weiter umfassend Befestigen einer Schneidklinge innerhalb der vertieften Fläche.

7. Verfahren nach Anspruch 6, weiter umfassend Einführen eines Füllmaterials in die vertiefte Fläche.

8. Verfahren nach Anspruch 7, wobei das Füllmaterial eines oder mehrere Materialien umfasst ausgewählt aus der Gruppe bestehend aus HDPE, Keramik, rostfreiem Stahl, UV-vernetzbarem Polyester und Nanopartikeln.

9. Verfahren nach Anspruch 1, weiter umfassend Aufbringen eines zweiten Materials auf die äußere Oberfläche des Ballons.

10. Verfahren nach Anspruch 9, wobei das zweite Material härter ist als das Material des Körper-Teilbereichs.

11. Verfahren nach Anspruch 10, wobei das Verfahren umfasst Ablatieren von Bereichen des zweiten Materials, um Schneidelemente zu bilden, die sich von der äußeren Oberfläche des Körper-Teilbereichs erstrecken.

12. Verfahren nach Anspruch 9, wobei das zweite Material weicher ist als das Material des Körper-Teilbereichs.

13. Verfahren nach Anspruch 12, wobei das Verfahren Entfernen zumindest eines Teilbereichs des zweiten Materials umfasst.

14. Verfahren nach Anspruch 13, wobei, nach Entfernen des Teilbereichs des zweiten Materials, das zweite Material in der Dicke zusammenläuft von einer ersten Endfläche zu einem zweiten Endfläche des Körper-Teilbereichs.

15. Verfahren nach einem der vorangehenden Ansprüche, wobei die zweiten Bereiche longitudinal platziert sind entlang des Körper-Teilbereichs in gleichmäßig beabstandeten Intervallen.

16. Verfahren nach Anspruch 1, wobei die äußere Oberfläche des Körper-Teilbereichs des Ballons eine durchschnittliche Rauheit von ungefähr 1 Mikrometer oder mehr hat.

17. Medizinische Vorrichtung mit einem Ballon (100; 200; 300; 400; 500; 600; 1200; 1300), umfassend einen ersten Kegel-Teilbereich und einen Körper-Teilbereich (102; 202; 302; 402; 502; 602; 1202; 1302);
wobei der Körper-Teilbereich des Ballons erste und zweite Bereiche aufweist, wobei die ersten Bereiche vertieft sind relativ zu den zweiten Bereichen,
wobei die ersten Bereiche Kanäle (108; 208; 308; 408; 508; 608; 1208; 1308) bilden, die die Dicke des Ballons reduzieren, wobei die Kanäle sich im Wesentlich longitudinal entlang des Ballons erstrecken, um als bevorzugte Falt-Teilbereiche zu dienen.

18. Medizinische Vorrichtung gemäß Anspruch 17, das weiter ein Füllmaterial in den vertieften Flächen umfasst.

19. Medizinische Vorrichtung gemäß Anspruch 18, wobei das Füllmaterial eine oder mehrere Materialien umfasst ausgewählt aus der Gruppe bestehend aus HDPE, Keramik, rostfreiem Stahl, UV-vernetzbarem Polyester und Nanopartikeln.

20. Vorrichtung gemäß Anspruch 17, weiter umfassend ein zweites Material auf der äußeren Oberfläche des Ballons.

21. Medizinische Vorrichtung gemäß Anspruch 20, wobei das zweite Material härter ist als das Material des Körper-Teilbereichs.

22. Medizinische Vorrichtung gemäß Anspruch 21, wobei das zweite Material weicher ist als das Material des Körper-Teilbereichs.

23. Medizinische Vorrichtung gemäß einem der Ansprüche 17 bis 22, wobei die zweiten Bereiche longitudinal platziert sind entlang des Körper-Teilbereichs in gleichmäßig beabstandeten Intervallen.

24. Medizinische Vorrichtung gemäß Anspruch 17, wobei die äußere Oberfläche des Körper-Teilbereichs des Ballons eine durchschnittliche Rauheit von ungefähr 1 Mikrometer oder mehr hat.

25. Medizinische Vorrichtung gemäß einem der vorangehenden Ansprüche, wobei die Kanäle (108; 208; 308; 408; 508; 608; 1208; 1308) gebildet werden, um eine vorherbestimmte Weise des Reißens zu ermöglichen.

## Revendications

1. Procédé, comprenant les opérations consistant à :
fournir un ballon médical (100 ; 200 ; 300 ; 400 ; 500 ; 600 ; 1200 ; 1300) comprenant une première portion en cône et une portion de corps (102 ; 202 ; 302 ; 402 ; 502 ; 602 ; 1202 ; 1302) ; et
enlever du matériau depuis une surface extérieure de la portion de corps du ballon pour former des premières régions et des secondes régions, les premières régions étant évidées par rapport aux secondes régions,
dans lequel les premières régions forment des canaux (108 ; 208 ; 308 ; 408 ; 508 ; 608 ; 1108 ; 1308) qui s'étendent généralement longitudinalement le long du ballon pour servir de portions de pliage préférentielles.

2. Procédé selon la revendication 1, dans lequel l'enlèvement de matériau depuis le ballon comprend de procéder une ablation du ballon au laser.

3. Procédé selon la revendication 2, dans lequel l'ablation du ballon au laser est effectuée avec une longueur d'onde d'environ 157 nm à environ 450 nm.

4. Procédé selon la revendication 3, dans lequel la longueur d'onde est environ 248 nm.

5. Procédé selon la revendication 3, dans lequel la longueur d'onde est environ 193 nm.

6. Procédé selon la revendication 1, comprenant en outre l'opération consistant à attacher une lame de coupe à l'intérieur de la zone évidée.

7. Procédé selon la revendication 6, comprenant en outre l'opération consistant à introduire un matériau de remplissage dans les zones évidées.

8. Procédé selon la revendication 7, dans lequel le matériau de remplissage comprend un ou plusieurs matériaux choisis parmi le groupe comprenant HDPE, céramique, acier inoxydable, polyester réticulable aux rayons UV, et nanoparticules.

9. Procédé selon la revendication 1, comprenant en outre l'opération consistant à appliquer un second matériau sur la surface extérieure du ballon.

10. Procédé selon la revendication 9, dans lequel le second matériau est plus dur que le matériau de la portion de corps.

11. Procédé selon la revendication 10, dans lequel le procédé comprend l'opération consistant à effectuer une ablation de régions du second matériau pour former des éléments de coupe s'étendant depuis la surface extérieure de la portion de corps.

12. Procédé selon la revendication 9, dans lequel le second matériau est plus tendre que le matériau de la portion de corps.

13. Procédé selon la revendication 12, dans lequel le procédé comprend l'opération consistant à enlever au moins une portion du second matériau.

14. Procédé selon la revendication 13, dans lequel, après avoir enlevé la portion du second matériau, le second matériau diminue en épaisseur depuis une première zone terminale vers une seconde zone terminale de la portion de corps.

15. Procédé selon l'une des revendications précédentes, dans lequel les secondes régions sont positionnées longitudinalement le long de la portion de corps à intervalles régulièrement espacés.

16. Procédé selon la revendication 1, dans lequel la surface extérieure de la portion de corps du ballon possède une rugosité moyenne d'environ 1 µm ou plus.

17. Dispositif médical ayant un ballon (100 ; 200 ; 300 ; 400 ; 500 ; 600 ; 1200 ; 1300) comprenant une première portion en cône et une portion de corps (102 ; 202 ; 302 ; 402 ; 502 ; 602 ; 1102 ; 1302) ;
dans lequel la portion de corps du ballon possède des premières régions et des secondes régions, les premières régions étant évidées par rapport aux secondes régions,
dans lequel les premières régions forment des canaux (108 ; 208 ; 308 ; 408 ; 508 ; 608 ; 1208 ; 1308) réduisant l'épaisseur du ballon, lesdits canaux s'étendant généralement longitudinalement le long du ballon pour servir de portions de pliage préférentielles.

18. Dispositif médical selon la revendication 17, comprenant en outre un matériau de remplissage dans les zones évidées.

19. Dispositif médical selon la revendication 18, dans lequel le matériau de remplissage comprend un ou plusieurs matériaux choisis parmi le groupe comprenant HDPE, céramique, acier inoxydable, polyester réticulable aux rayons UV, et nanoparticules.

20. Dispositif médical selon la revendication 17, comprenant en outre un second matériau sur la surface extérieure du ballon.

21. Dispositif médical selon la revendication 20, dans lequel le second matériau est plus dur que le matériau de la portion de corps.

22. Dispositif médical selon la revendication 21, dans lequel le second matériau est plus tendre que le matériau de la portion de corps.

23. Dispositif médical selon l'une des revendications 17 à 22, dans lequel les secondes régions sont positionnées longitudinalement le long de la portion de corps à intervalles régulièrement espacés.

24. Dispositif médical selon la revendication 17, dans lequel la surface extérieure de la portion de corps du ballon possède une rugosité moyenne d'environ 1 µm ou plus.

25. Dispositif médical selon l'une des revendications précédentes, dans lequel les canaux (108 ; 208 ; 308 ; 408 ; 508 ; 608 ; 1208 ; 1308) sont formés pour faciliter un mode de rupture prédéterminé.
